# EUROPEAN PATENT SPECIFICATION

(11) **EP 0 540 813 B1**
(45) Date of publication and mention of the grant of the patent: **20.03.1996**
(21) Application number: 92108329.1
(22) Date of filing: 18.05.1992
(51) Int. Cl.: A61K 9/20, A61K 31/425

(54) **Sustained release formulations of acetazolamide**
Acetazolamide Formulierung mit verzögerter Wirkstoffabgabe
Formulations d'acétazolamide à libération retardée

(30) Priority: 11.06.1991 GB 9112495
(43) Date of publication of application: 12.05.1993
(73) Proprietor: AMERICAN CYANAMID COMPANY, Wayne New Jersey 07470-8426 (US)
(72) Inventor: Payne, Nicholas Ian, Southampton SO3 7DP, Hampshire (GB); Parker, David, Southampton SO3 2JS, Hampshire (GB); Potts, Angela Catherine, Fareham PO14 2RT, Hampshire (GB); Staines, Laurence Charles, Fareham PO16 8SZ, Hampshire (GB)
(74) Representative: Wileman, David Francis, Dr.

(56) References cited:
- EP-A- 0 223 365
- EP-A- 0 418 597
- GB-A- 2 135 879
- US-A- 3 671 633
- BIOLOGICAL ABSTRACT Number 68024183 Philadelphia, P.A., US. SCHOENWALD R.D. et al.: "Decreased bio-availability of sustained release acetazo lamide dosage forms."
- BIOLOGICAL ABSTRACT Number: 70053249 Philadelphia, P.A., US; BERSON F.G. et al. : " Acetazolamide dosage forma in the treatment of glaucoma."

## Description

This invention relates to an improved sustained release formulation of the drug acetazolamide, and to a method for preparing such formulations.

Acetazolamide, or N-(5-sulphamoyl-1,3,4-thiadiazol-2-yl)acetamide to give its full name, is an effective ocular hypotensive agent when it is administered systemically, and it is widely used for the treatment of glaucoma, in certain convulsive disorders and in situations of abnormal fluid retention, for example cardiac oedema.

However, the potential therapeutic value of acetazolamide is sometimes limited by side effects and poor patient compliance when the drug is administered in tablet form. This situation has led to the availability of a sustained release dosage form of acetazolamide which is designed to maintain a relatively constant drug level in the blood, over a relatively long period, thereby avoiding the peaks of drug concentration usually associated with tablet dosing and hence reducing the incidence of concentration levels of the drug liable to cause side effects. The sustained release formulation also permits the dosing frequency to be reduced to twice daily, whereby patient compliance with the prescribed dosage can be improved.

Currently, the sustained release formulation of acetazolamide which we manufacture consists of small wax-coated granules of the drug encapsulated in a soft gelatin capsule. The granules themselves are formed by a conventional granulation technique using gelatin as a binder to hold the particles of drug powder together, and then the coating of wax, which serves to control the in vivo rate of release of the drug by diffusion is applied by spraying. However, as the granules themselves are inevitably of irregular shape, the applied wax coating tends to be of uneven thickness, which of course in turn affects the drug-release properties of the coating. Now the desired sustained drug release profile is obtained by mixing together different batches with different thicknesses of wax coating, but because of the difficulty referred to of ensuring evenly coated granules it is actually, in practice, difficult to ensure that the desired release profile is consistently obtained.

The need for sustained release formulation of acetazolamide which can be more efficiently and more consistently produced is apparent.

Modern extrusion/spheronisation technology provides a means for obtaining small, regular spheres of a drug and therefore offered the prospect of achieving an even coating of wax or other release-controlling membrane on acetazolamide, whereby the desired sustained release profile could be obtained consistently and reliably with this drug.

In brief, an extrusion spheronisation process typically involves the following steps:
(i) form a wet, extendable blend of the drug and required excipients,
(ii) extrude the blend into small, cylindrical pellets, and
(iii) roll the pellets in a spheronizer, which in essence consists of a rotating disc at the base of a short stationary cylinder, whereby the pellets are broken up and formed into small, regular spheres. The size of the resulting spherical pellets can be controlled by appropriate selection of such operational parameters of the spheronizer as disc speed, residence time in the spheronizer, spheronizer size and extrudate dimensions.

We therefore investigated the possibility of using the extrusion/spheronisation process to obtain spherical particles of acetazolamide for coating with wax or other release-controlling membrane. To our surprise, we found that we were quite unable to form satisfactory spherical particles of acetazolamide by this technique when we prepared for extrusion a blend of the drug and the usual excipients. In a conventional extrusion/spheronisation process for producing pellets of a drug, the drug is mixed with a binder such as a gum or sugar, a moisture-control agent such as microcrystalline cellulose and water so as to form a mass having the consistency of dry dough which can be extruded into small cylindrical pellets ready for spheronisation. The binder serves to hold the drug and the other excipients together during spheronisation and thus facilitate high loadings of drug, while the moisture control agent serves the dual purpose of making the water available to plasticize the blend thereby to facilitate the extrusion but then, during spheronisation, releases the water in a slow, controlled manner whereby the spherical pellets are not disrupted by too rapid drying.

After considerable experimentation we found that, although we desired a high loading of acetazolamide, upwards of 75%, in order to achieve a final dosage form of a reasonable size for oral administration, nonetheless the presence of a binder was a prime cause of the difficulties which we were experiencing. This discovery was quite contrary to conventional practice which teaches that a binder component is essential particularly when drug loadings of the level which we were using are required.

It is possible that the binder was in some way interfering to prevent the moisture control agent from acting in the desired way, and also it appeared that the binder was tending to stick the forming spheres together whereby large agglomerates resulted.

Based on this experimental work, we have now developed a new sustained release formulation of acetazolamide.

We have found that the acetazolamide itself acts as its own binder to enable the desired high drug loading to be achieved. We postulate that a small proportion of the acetazolamide dissolves during the wet blending step to form "liquid bridges" between the particles. On drying, removal of the water will leave interparticulate bonds within the structure of the pellets, whereby the pellets retain their coherency.

Thus, the present invention provides a new sustained release formulation of acetazolamide which comprises substantially spherical, essentially binder-free pellets of not more than 0.1% by weight of a binder or totally absent containing at least 25% by weight of acetazolamide, preferably from 60% to 85% by weight, most preferably from 79% to 82% by weight, said pellet being individually coated with a release-controlling membrane.

The present invention also provides a method for preparing a sustained release formulation of acetazolamide, comprising the steps of:
(a) forming, essentially binder-free pellets of not more than 0.1% by weight of a binder or totally absent containing acetazolamide;
(b) spheronising said acetazolamide pellets, and
(c) coating the resulting substantially spherical acetazolamide pellets with a release-controlling membrane.

The acetazolamide-containing pellets of the present invention can be filled into soft or hard gelatin capsules or otherwise presented in a unit dosage form for administration to a patient.

In addition to being obtainable by the more efficient, more reproducible extrusion/spheronisation technique, the present acetazolamide-containing pellets are found to have excellent bioavailability and release characteristics. Indeed, by means of the present invention we have been able to produce pellets whose release and bioavailability characteristics are such as to provide a unit dosage form permitting once-a-day dosing of acetazolamide, rather than the twice-a-day dosing considered necessary with the present granular sustained-release formulation.

The pellets of acetazolamide suitably have a particle size of 500-1250 µm (microns) prior to coating, preferably a particle size of 800-1000 µm (microns). As indicated above, the particle size is mainly controlled by selection of the spheronizer and its operating parameters, but nonetheless it is good practice to sieve the spheronizer pellets in order to remove the, usually small, proportion of fines and agglomerated particles. The pellets are, as previously noted, substantially spherical, thereby facilitating an even coating of the release-controlling membrane.

In addition to the active ingredient, the spherical pellets of the present invention will generally also contain a moisture-controlling agent and a surfactant, these being ingredients usually necessary to achieve proper moisture-control for satisfactory spheronisation. However, no separate binder needs to be included, and indeed such binder should be essentially absent, not more than 0.1% by weight, and preferably totally absent. Although not normally preferred since it reduces drug loading, other ingredients such as hydrophilic agents, e.g. polyethylene glycol can optionally be included in the pellets if desired.

As the moisture control agent, we generally prefer to use a microcrystalline cellulose, such as those obtainable under the trade mark "Avicel" from FMC Corporation. The surfactant is preferably sodium lauryl sulphate, but other ionic and non-ionic surface active agents are also suitable.

In order to achieve the desired sustained-release properties, the spherical pellets are coated with a release-controlling membrane, such as wax as in the present granular formulation or one of the commercially available polymeric release-control materials such as those based on cellulose derivatives e.g. ethylcellulose, ethylcellulose latex, hydroxypropyl methylcellulose, or acrylic resins e.g. combined polymers of methacrylic acid and methacrylic acid esters, or mixtures thereof. We currently prefer to use a mixture of a water-insoluble film former such as ethylcellulose and a water-soluble film former such as hydroxypropyl methylcellulose to form a membrane which in vivo will develop pores which allow diffusion of the acetazolamide from the core at a controlled rate, which depends on the relative proportions of the two cellulose derivatives in the membrane. The release-control coating may be applied by any of the techniques conventionally used in the art, preferably to a thickness of 5 to 50 µm (microns), more usually 20 to 30 µm (microns).

Although the acetazolamide-containing pellets of the present invention are generally made by conventional extrusion/spheronisation procedures (apart, of course, from the omission from the blend of a separate binder), we have found that spheronisation proceeds more satisfactorily, particularly with higher drug loadings i.e. above 50% by weight, if the blending of the wet mass prior to spheronisation and extrusion is conducted under high shear. It is considered that the application of the high shear helps to dissolve a small amount of the acetazolamide in the water, whereby it can then serve as the binder as described above, and moreover the high shear may also help the moisture control agent to function better. In any event, in the absence of high shear during the blending step we have found it difficult to adequately control moisture levels whereby spherical pellets of desired characteristics can be repeatedly obtained without intermediate drying of the equipment. Indeed, with very high drug loadings (of the order of 80% by weight) we have found that the application of high shear during the blending is probably essential and certainly strongly recommended.

A preferred procedure for making acetazolamide-containing pellets in accordance with the present invention is as follows:

| Composition of Pellet Core | | |
|---|---|---|
| Ingredient | % Composition, dry basis | |
| | General | Preferred |
| acetazolamide | 65-85 | 80-82 |
| microcrystalline cellulose | 15-35 | 18-20 |
| surfactant water* | 0-0.25 | 0.04-0.08 |
| binder | absent | |

| | | |
|---|---|---|
| *removed during drying stage | | |

| Composition of Film Coating Solution | | |
|---|---|---|
| ethylcellulose | 0.5-1.5 | 0.65-0.8 |
| hydroxy propylmethyl cellulose | 1-4 | 2.5-3.0 |
| plasticizer | 0.1-1.0 | 0.3-0.5 |
| liquid carrier | to 100% | to 100% |

### Step 1

Dry blend the acetazolamide powder and microcrystalline cellulose.

### Step 2

Add the surfactant and water to the dry blend.

### Step 3

Blend the mixture to form an extrudable mass of dry dough i.e. so that it is mouldable in the hand but breaks to give a rough fractured surface. For at least a proportion of the wet blending, and preferably throughout, apply high shear, meaning use a high shear blender, namely a blender having the following characteristics, viz:
(1) The material is propelled in two or more planes by two or more mixing elements. It is the interaction and working of these planes of movement against one another which results in the high shear mixing action. (Low shear blenders usually only have one mixing element and in consequence work in one plane only.)
(2) The tip speed of the mixing elements is at least 5 metres per second (low shear mixers have tip speeds of the order of 1 metre per second).

### Step 4

Extrude the wet blend to form cylindrical pellets, typically of from 1 to 40 mm long, with the majority being 10 mm long, and with a diameter of 0.4 to 2 mm, usually 0.8 mm.

### Step 5

Roll the pellets in a spheronizer.

As a result of the rolling motion produced within the spheronizer, the rods first break up into shorter lengths (in the order of 1 mm long), and these particles then roll over one another and become rounded off to yield substantially spherical pellets with a relatively tight size distribution. Further the centrifugal forces generated within the spheronizer compress the pellets which in consequence are densified.

The spheronizer is operated so that the pellets produced are predominantly within the range of 500-1250 µm (microns).

### Step 6

Dry the wet spherical pellets in an oven, preferably to a moisture content of from 0.5 to 1.5%. Lower moisture contents can present static handling problems.

### Step 7

Screen the spherical pellets to obtain a batch size of 500-1250 microns.

### Step 8

Prepare a dispersion of the ethylcellulose, hydroxypropyl methyl cellulose and plasticizer in the liquid carrier.

### Step 9

Coat the pellets obtained in step (7) with the release coating formed in step (8), by spray application using a fluid bed system fitted with, for example, a Wurster column.

### Step 10

Dry the film-coated pellets.

Preferably, the resulting sustained-release acetazolamide pellets are filled into hard gelatin capsules, either 250 mg or 500 mg per capsule. The 500 mg capsule can provide sufficient drug to permit once-daily dosing, and indeed in some instances the 250 mg capsule can do so as well.

The invention is illustrated by the Examples which follow:

### Example 1

This example describes the preparation of the currently preferred coated pellets of the present invention, having the following composition:

| Composition of pellet core | |
|---|---|
| Ingredient | % Composition, dry basis |
| acetazolamide | 80.0 |
| microcrystalline cellulose (Avicel PH101) | 19.94 |
| sodium lauryl sulphate | 0.06 |

| Composition of Coated Pellets | |
|---|---|
| Ingredient | % Composition, dry basis |
| acetazolamide pellets | 94.55 |
| ethylcellulose (10cps) | 0.72 |
| hydroxypropylmethyl cellulose (6cps) | 2.86 |
| mineral oil* | 0.41 |
| colourant** | 1.46 |

| | |
|---|---|
| * plasticizer | |
| **Opaspray K-I-2506, giving an orange colour | |

First, the preparation of the acetazolamide pellet cores will be described.

Acetazolamide raw material was first milled to break up aggregates. A weighed amount (8000 g) of the milled acetazolamide powder and the Avicel PH101 (1994 g) were now weighed into a high shear blender (Pharma Matrix PMA 65/25/2G, manufactured by T.C. Fielder) having two mixing blades, one larger, one smaller, working in two planes at 90° to one another. The blender was operated for 5 minutes with the tip speed of the larger mixing element being 5.3 m/sec, and that of the smaller element being at the higher of its two operating speeds (corresponding to a tip speed of 22 m/sec) to ensure thorough mixing of the acetazolamide and microcrystalline cellulose. Then the sodium lauryl sulphate (6 g) dissolved in 4700 ml of purified water was slowly added, the high shear blender was again operated for 10 minutes but this time with blade tip speed of the larger mixing element being increased to 10.6 m/sec, the speed of the smaller mixing element being kept at its higher setting.

The wet mass obtained was now extruded through an extruder (E140.4, manufactured by Nica Systems, Sweden) fitted with a mesh of 0.8 mn, thereby forming cylindrical pellets whose diameter was 0.8 mn and having lengths ranging from 1 to 40 mn, but with the majority of pellets being about 10 mm long.

The extruded pellets were now placed in a spheronizer (S-320, Nica Systems, Sweden) which was operated for two minutes at approximately 800 rpm. The result of this spheronisation was to form substantially spherical pellets with a size predominantly in the range 500-1250 µm (microns). These pellets were now dried in a hot air oven for 8 hours at 50°C, to reduce the residual moisture level to about 1%, and the dried pellets were then screened through a 14 mesh and 25 mesh (British Standard). The pellets obtained lay within the size range 500-1250 µm (microns).

Next the preparation of the release coating solution will be described.

Methanol (320 ml) was measured into a mixing vessel, and then the hydroxypropylethyl cellulose (28.6 g) and the ethylcellulose (7.2 g) were stirred in by means of a paddle stirrer. When complete dispersion had been achieved, mineral oil (4.1 g) and the colourant (29.74 g, containing 49.1% solids) were added to the dispersion. Finally, methylene chloride (1010 ml) was added to the dispersion and thoroughly mixed in.

The substantially spherical acetazolamide pellets (945.5 g) were now coated with the release coating material to form the release membrane. This was achieved in a fluidized bed. The coating material was sprayed on at the rate of 40 ml per minute. Finally, 200 ml of a methanol/methylene chloride mixture (1:3 by volume) was sprayed on to give the pellets a gloss, and the pellets were dried in a current of warm air at approximately 60°C.

The resulting coated pellets had a coating thickness of approximately 1 mg per square centimetre of pellet surface area.

### Comparative Example 1

Acetazolamide raw material was milled using an apex mill fitted with a 0.027 x 2.54 x 10⁻²m (0.027 inch) nominally rated screen. This served to break up aggregates and provide a uniform starting material.

800 g of the acetazolamide were then mixed with 199.4 g of Avicel PH 101 using either a low shear blender (Kenwood Planetary Mixer) or a high shear blender (Pharma Matrix PMA 65/25/2G).

0.006 g of sodium lauryl sulphate was dissolved in 300 ml water, and 10 ml of a 7.5% aqueous gelatin solution (binder solution) were added. The resulting solution was then slowly added to the acetazolamide/Avicel mixture and mixed in for 10 minutes, in a manner analogous to that of Example 1, using a high speed for both the low shear and high shear blenders. The resulting mass was then extruded, and spheronisation attempted.

The extrusion/spheronisation procedure was repeated after the addition of each of 2 x 100 ml additional aliquots of water.

The experiment was also repeated but using 50 ml, 100 ml or 200 ml of the 7.5% gelatin solution.

In these experiments it was found that at the higher moisture levels larger agglomerates were formed in the spheronizer, whilst at lower levels the spheroids were broken down by attrition into fines. In no instance was spheronisation satisfactorily achieved.

This experiment demonstrates that the presence of the gelatin binder prevented satisfactory spheronisation even when the high shear blender was employed

No attempt was made to coat the rough pellets or fines resulting from these experiments due to their wholly unacceptable nature.

### Example 2

A bioavailability study was carried out in ten elderly volunteers), 7 males and 3 females, aged 60-74 years. The volunteers were divided into two groups, A and B. The volunteers in Group A took one conventional 250 mg acetazolamide tablet every 12 hours, whilst those in Group B took two 250 mg acetazolamide capsules containing coated pellets prepared as described in Example 1 together once every 24 hours. Dosing was started at 10 pm on Day 1 and blood samples were taken, to determine the plasma concentration profile under steady state conditions, at the following times after the 10 pm dose on Day 9:

### Conventional Tablet

0.5, 1, 2, 3, 4, 6, 9, 12 hours and at same times after 10 am on Day 10.

### Formulation of Invention

1, 2, 3, 4, 6, 9, 12, 16 and 24 and at same times after 10 am dose on Day 10.

After an eleven day washout period, without drug administration, the two groups were crossed over, with Group A taking two formulations of this invention together once every 24 hours and Group B taking one conventional tablet every 12 hours. The blood sampling was repeated as before.

The levels of acetazolamide were determined by HPLC in the separated plasma.

A similar study was also carried out in eight healthy male volunteers, aged 19-27 years.

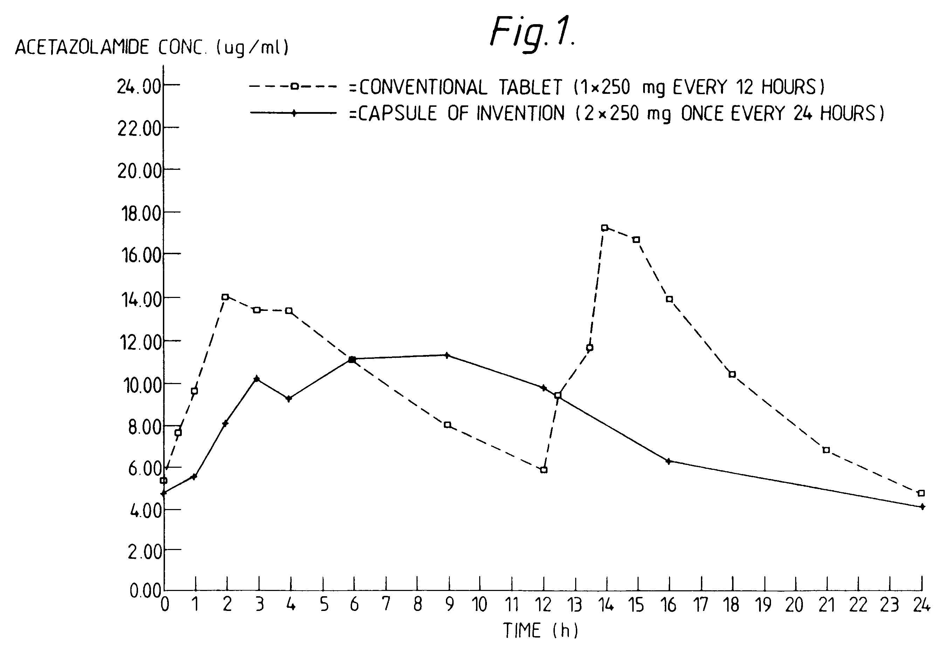

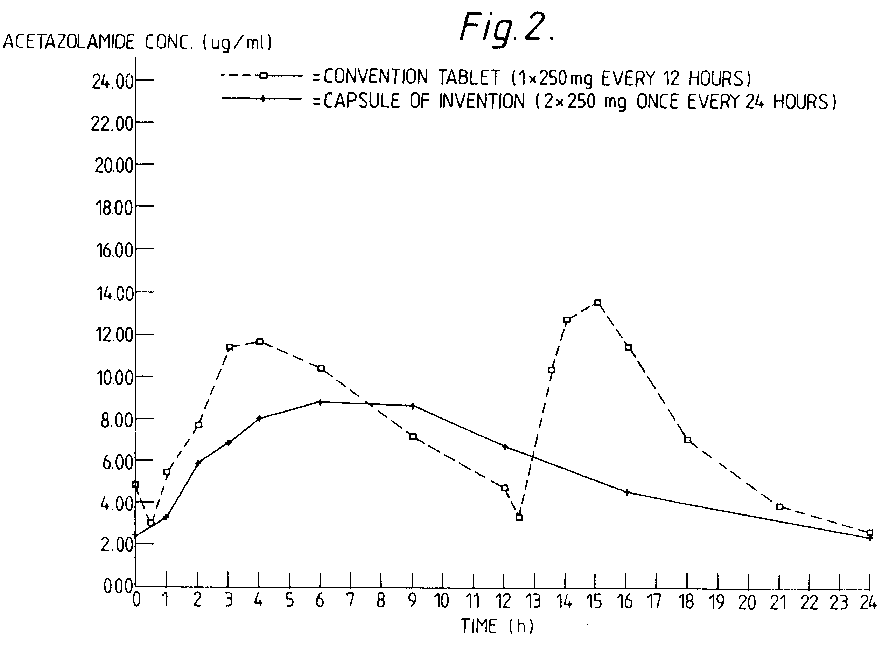

The results of this study are graphically shown in Figs. 1 and 2 of the accompanying drawings, Fig. 1 showing the mean acetazolamide plasma concentration plotted against time for the study in elderly patients, and Fig. 2 showing the same information for the study in young, healthy male volunteers.

It has been reported that acetazolamide has a therapeutic plasma range of 5-10 µg/ml, with levels above 30 µg/ml being considered toxic. It will be seen from Figs. 1 and 2 that with both groups of patients, 2 x 250 mg capsules dosed once per day were sufficient to maintain the plasma concentration of acetazolamide within the therapeutically effective range. In contrast, with the conventional acetazolamide tablet formulation the plasma levels reached a higher peak but thereafter fell rapidly to below the therapeutically effective range, making it necessary to administer a second tablet in each 24 hours in order to maintain efficacy.

## Claims (Claims for the following Contracting State(s): AT, BE, CH, LI, DE, DK, ES, FR, GR, IT, LU, NL, PT, SE)

1. A sustained release formulation of acetazolamide, comprising substantially spherical, essentially binder-free pellets having not more than 0.1% by weight of a binder or being totally binder free and containing at least 25% by weight of acetazolamide, said pellets being coated with a release-controlling membrane.

2. An acetazolamide formulation according to Claim 1, wherein said pellets contain from 60 to 85% by weight of acetazolamide.

3. An acetazolamide formulation according to Claim 2, wherein said pellets contain from 79 to 82 % by weight of acetazolamide.

4. An acetazolamide formulation according to any preceding claim, wherein said pellets also comprise a moisture-controlling agent and/or a surfactant.

5. An acetazolamide formulation according to Claim 4, wherein said moisture-controlling agent is microcrystalline cellulose.

6. An acetazolamide formulation according to Claim 4 or Claim 5, wherein said surfactant is sodium lauryl sulphate.

7. An acetazolamide formulation according to any preceding claim, wherein said release controlling membrane comprises a mixture of water-soluble film-forming substance and a water-insoluble film-forming substance.

8. An acetazolamide formulation according to Claim 7, wherein said release controlling membrane comprises hydroxypropylmethyl cellulose and ethyl cellulose.

9. An acetazolamide formulation according to any preceding claim presented in a unit dosage form.

10. A method for preparing a sustained release formulation of acetazolamide, comprising the steps of:
(a) forming, essentially binder-free pellets having not more than 0.1% by weight of a binder or being totally binder free and containing acetazolamide;
(b) spheronising said acetazolamide pellets; and
(c) coating the resulting substantially spherical acetazolamide pellets with a release-controlling membrane.

## Claims (Claims for the following Contracting State(s): ES, GR)

1. A method for preparing a sustained release formulation of acetazolamide, comprising the steps of:
(a) forming essentially binder-free pellets having not more than 0.1% by weight of a binder or being totally binder free and containing at least 25% by weight of acetazolamide;
(b) spheronising said acetazolamide pellets; and
(c) coating the resulting substantially spherical acetazolamide pellets with a release-controlling membrane.

2. A process according to Claim 1, wherein said pellets contain from 60 to 85% by weight of acetazolamide.

3. A process according to Claim 2, wherein said pellets contain from 79 to 82% by weight of acetazolamide.

4. A process according to any one of the preceding claims, wherein said pellets also comprise a moisture-controlling agent and/or a surfactant.

5. A process according to Claim 4 wherein said moisture-controlling agent is microcrystalline cellulose.

6. A process according to Claim 4 or Claim 5, wherein said surfactant is sodium lauryl sulphate.

7. A process according to any preceding claim, wherein said release controlling membrane comprises a mixture of water-soluble film-forming substance and a water-insoluble film-forming substance.

8. A process according to Claim 7, wherein said release controlling membrane comprises hydroxypropylmethyl cellulose and ethyl cellulose.

9. A process according to any preceding claim in which the formulation is presented in a unit dosage form.

## Patentansprüche (Patentansprüche für folgende(n) Vertragsstaat(en): AT, BE, CH, LI, DE, DK, ES, FR, GR, IT, LU, NL, PT, SE)

1. Acetazolamid-Formulierung mit Langzeit-Freisetzung, welche im wesentlichen sphärische, im wesentlichen Bindemittel-freie Pellets, die nicht mehr als 0,1 Masse-% eines Bindemittels enthalten oder völlig Bindemittel-frei sind, und die zumindest 25 Masse-% Acetazolamid enthalten, umfaßt, wobei die Pellets mit einer Freisetzungsregulierungsmembran überzogen sind.

2. Acetazolamid-Formulierung nach Anspruch 1, worin die Pellets 60 bis 85 Masse-% Acetazolamid enthalten.

3. Acetazolamid-Formulierung nach Anspruch 2, worin die Pellets 79 bis 82 Masse-% Acetazolamid enthalten.

4. Acetazolamid-Formulierung nach einem der vorhergehenden Ansprüche, worin die Pellets auch ein Feuchtigkeitsregulierungsmittel und/oder einen grenzflächenaktiven Stoff umfassen.

5. Acetazolamid-Formulierung nach Anspruch 4, worin das Feuchtigkeitsregulierungsmittel mikrokristalline Cellulose ist.

6. Acetazolamid-Formulierung nach Anspruch 4 oder 5, worin der grenzflächenaktive Stoff Natriumlaurylsulfat ist.

7. Acetazolamid-Formulierung nach einem der vorhergehenden Ansprüche, worin die Freisetzungsregulierungsmembran eine Mischung einer wasserlöslichen Filmbildnersubstanz und einer wasserunlöslichen Filmbildnersubstanz umfaßt.

8. Acetazolamid-Formulierung nach Anspruch 7, worin die Freisetzungsregulierungsmembran Hydroxypropylmethylcellulose und Ethylcellulose umfaßt.

9. Acetazolamid-Formulierung nach einem der vorhergehenden Ansprüche, welche in Einheitsdosierungsform dargereicht wird.

10. Verfahren zur Herstellung einer Acetazolamid-Formulierung mit Langzeit-Freisetzung, welches die Schritte umfaßt:
(a) Bilden im wesentlichen Bindemittel-freier Pellets, die nicht mehr als 0,1 Masse-% eines Bindemittels enthalten oder völlig Bindemittel-frei sind, und die Acetazolamid enthalten;
(b) Formen der Acetazolamid-Pellets zu Kugeln; und
(c) Überziehen der erhaltenen, im wesentlichen sphärischen Acetazolamid-Pellets mit einer Freisetzungsregulierungsmembran.

## Patentansprüche (Patentansprüche für folgende(n) Vertragsstaat(en): ES, GR)

1. Verfahren zur Herstellung einer Acetazolamid-Formulierung mit Langzeit-Freisetzung, welches die Schritte umfaßt:
(a) Bilden im wesentlichen Bindemittel-freier Pellets, die nicht mehr als 0,1 Masse-% eines Bindemittels enthalten oder völlig Bindemittel-frei sind, und die zumindest 25 Masse-% Acetazolamid enthalten;
(b) Formen der Acetazolamid-Pellets zu Kugeln; und
(c) Überziehen der erhaltenen, im wesentlichen sphärischen Acetazolamid-Pellets mit einer Freisetzungsregulierungsmembran.

2. Verfahren nach Anspruch 1, bei welchem die Pellets 60 bis 85 Masse-% Acetazolamid enthalten.

3. Verfahren nach Anspruch 2, bei welchem die Pellets 79 bis 82 Masse-% Acetazolamid enthalten.

4. Verfahren nach einem der vorhergehenden Ansprüche, bei welchem die Pellets auch ein Feuchtigkeitsregulierungsmittel und/oder einen grenzflächenaktiven Stoff umfassen.

5. Verfahren nach Anspruch 4, bei welchem das Feuchtigkeitsregulierungsmittel mikrokristalline Cellulose ist.

6. Verfahren nach Anspruch 4 oder 5, bei welchem der grenzflächenaktive Stoff Natriumlaurylsulfat ist.

7. Verfahren nach einem der vorhergehenden Ansprüche, bei welchem die Freisetzungsregulierungsmembran eine Mischung einer wasserlöslichen Filmbildnersubstanz und einer wasserunlöslichen Filmbildnersubstanz umfaßt.

8. Verfahren nach Anspruch 7, bei welchem die Freisetzungsregulierungsmembran Hydroxypropylmethylcellulose und Ethylcellulose umfaßt.

9. Verfahren nach einem der vorhergehenden Ansprüche, bei welchem die Formulierung in Einheitsdosierungsform dargereicht wird.

## Revendications (Revendications pour l'(les) Etat(s) contractant(s) suivant(s): AT, BE, CH, LI, DE, DK, ES, FR, GR, IT, LU, NL, PT, SE)

1. Formulation à libération étalée d'acétazolamide, comprenant des pastilles sensiblement sphériques, essentiellement exemptes de liant, à savoir ne contenant pas ou pas plus de 0,1% en poids d'un liant, contenant au moins 25% en poids d'acétazolamide, les pastilles étant enduites d'une membrane de contrôle de la libération.

2. Formulation d'acétazolamide suivant la revendication 1, dans laquelle les pastilles contiennent de 60 à 85% en poids d'acétazolamide.

3. Formulation d'acétazolamide suivant la revendication 2, dans laquelle les pastilles contiennent de 79 à 82% en poids d'acétazolamide.

4. Formulation d'acétazolamide suivant l'une quelconque des revendications précédentes, dans laquelle les pastilles comprennent également un agent contrôlant l'humidité et/ou un tensioactif.

5. Formulation d'acétazolamide suivant la revendication 4, dans laquelle l'agent de contrôle de l'humidité est la cellulose microcristalline.

6. Formulation d'acétazolamide suivant la revendication 4 ou 5, dans laquelle le tensioactif est le laurylsulfate de sodium.

7. Formulation d'acétazolamide suivant l'une quelconque des revendications précédentes, dans laquelle la membrane contrôlant la libération comprend un mélange d'une substance filmogène soluble dans l'eau et d'une substance filmogène insoluble dans l'eau.

8. Formulation d'acétazolamide suivant la revendication 7, dans laquelle la membrane contrôlant la libération comprend de l'hydroxypropylméthylcellulose et de l'éthylcellulose.

9. Formulation d'acétazolamide suivant l'une quelconque des revendications précédentes, présentée sous une forme de dosage unitaire.

10. Procédé de préparation d'une formulation à libération étalée d'acétazolamide, comprenant les étapes de :
(a) formation de pastilles essentiellement exemptes de liant, à savoir ne contenant pas ou pas plus de 0,1% en poids d'un liant, contenant de l'acétazolamide;
(b) sphéroïdation des pastilles d'acétazolamide, et
(c) enduction des pastilles d'acétazolamide résultantes sensiblement sphériques au moyen d'une membrane contrôlant la libération.

## Revendications (Revendications pour l'(les) Etat(s) contractant(s) suivant(s): ES, GR)

1. Procédé de préparation d'une formulation à libération étalée d'acétazolamide, comprenant les étapes de :
(a) formation de pastilles essentiellement exemptes de liant, à savoir ne contenant pas ou pas plus de 0,1% en poids d'un liant, contenant de l'acétazolamide;
(b) sphéroïdation de pastilles d'acétazolamide, et
(c) enduction des pastilles résultantes d'acétazolamide sensiblement sphériques au moyen d'une membrane contrôlant la libération.

2. Procédé suivant la revendication 1, dans lequel les pastilles contiennent de 60 à 85% en poids d'acétazolamide.

3. Procédé suivant la revendication 2, dans lequel les pastilles contiennent de 79 à 82% en poids d'acétazolamide.

4. Procédé suivant l'une quelconque des revendications précédentes, dans lequel les pastilles comprennent également un agent contrôlant l'humidité et/ou un tensioactif.

5. Procédé suivant la revendication 4, dans laquelle l'agent contrôlant l'humidité est la cellulose microcristalline.

6. Procédé suivant la revendication 4 ou 5, dans lequel le tensioactif est le laurylsulfate de sodium.

7. Procédé suivant l'une quelconque des revendications précédentes, dans lequel la membrane contrôlant la libération comprend un mélange d'une substance filmogène soluble dans l'eau et d'une substance filmogène insoluble dans l'eau.

8. Procédé suivant la revendication 7, dans lequel la membrane contrôlant la libération comprend de l'hydroxypropylméthylcellulose et de l'éthylcellulose.

9. Procédé suivant l'une quelconque des revendications précédentes, dans lequel la formulation est présentée sous une forme de dosage unitaire.
